# EUROPEAN PATENT SPECIFICATION

(11) **EP 2 022 387 B1**
(45) Date of publication and mention of the grant of the patent: **04.07.2012**
(21) Application number: 07743658.2
(22) Date of filing: 18.05.2007
(51) Int. Cl.: A61B 1/00, G02B 23/24

(54) **ENDOSCOPE AND ENDOSCOPE SYSTEM**
ENDOSKOP UND ENDOSKOP-SYSTEM
ENDOSCOPE ET SYSTEME D'ENDOSCOPE

(30) Priority: 31.05.2006 JP 2006152598
(43) Date of publication of application: 11.02.2009
(73) Proprietor: Olympus Medical Systems Corp., Tokyo 151-0072 (JP)
(72) Inventor: ICHIMURA, Hironobu, Shibuya-ku Tokyo 151-0072 (JP); TAKATO, Hideyasu, c/o OLYMPUS MED. SYSTEMS CORP., Tokyo 151-0072 (JP); NOGUCHI, Azusa, c/o OLYMPUS MEDICAL SYSTEMS CORP., Tokyo 151-0072 (JP); MIYAKE, Kiyoshi, c/o OLYMPUS MEDICAL SYSTEMS CORP., Tokyo 151-0072 (JP); DOGUCHI, Nobuyuki, c/o OLYMPUS MED. SYSTEMS CORP., Tokyo 151-0072 (JP); GONO, Kazuhiro ,c/o OLYMPUS MEDICAL SYSTEMS CORP., Tokyo 151-0072 (JP)
(74) Representative: von Hellfeld, Axel
(86) International application number: PCT/JP2007/060223
(87) International publication number: WO 2007/138889

(56) References cited:
- JP-A- 03 289 769
- JP-A- 04 082 532
- JP-A- 07 222 712
- JP-A- 2003 153 849
- JP-A- 2005 230 183
- US-A- 5 178 130
- US-A1- 2005 197 530
- US-A1- 2005 228 452

## Description

### Technical Field

The present invention relates to an endoscope and an endoscope system which can observe a subject with an observation portion abutting on or spaced from the subject.

### Background Art

Conventionally, Japanese Patent Application Laid-Open Publication No. 2004-350940 has disclosed an endoscope having an observation probe which can observe a subject at high magnification with a distal end of an observation portion abutting on or spaced from the subject. This endoscope is provided with both a normal observation portion having normal magnification at an insertion distal end portion and a high magnification observation probe having high magnification which can appear and disappear from a distal end face of an insertion portion. According to this endoscope, after making observation by means of the normal observation portion to find a diseased region, the diseased region can be observed with magnification or photographed with magnification by means of the high magnification observation probe, for example.

However, the endoscope (probe main body) disclosed in the above-described Japanese Patent Application Laid-Open Publication No. 2004-350940 is inserted into a treatment instrument insertion hole of another endoscope main body to make observation, and therefore has a distal end portion of small diameter. Therefore, when the distal end portion abuts on a body wall forcibly, localized force tends to be applied because of a small abutment area. In addition, the distal end face of the high magnification observation probe needs to be positioned at a predetermined position within a near distance side observation depth of the normal observation portion. In the above-described endoscope, however, the high magnification observation probe is small, when the body wall is observed with magnification, gripping force is so small that there is a possibility of displacement in a direction perpendicular to the observation optical axis.

US 2005/0228452, on which document the preamble of claim 1 is based, discloses an apparatus for treating tissue including a flexible catheter including: a proximal end, a distal end for introduction into a chamber of a heart, a transparent balloon carried by the distal end, an optical imaging assembly carried by the distal end for imaging tissue structures beyond the distal end through the balloon, and a needle deployable from the tubular member for penetrating the tissue structure to treat tissue. A proximal end of the balloon is fixed on an outer surface of the catheter, and a distal end of the balloon is fixed around an outer surface of a treatment endoscope that is passed through a lumen of the catheter such that a distal end of the treatment endoscope opens to the outside. Tools such as a needle may be passed through the treatment endoscope to treat a target tissue area. When the treatment endoscope is advanced distally, the balloon is expanded by adding a substantially transparent material to the inside of the balloon. The optical imaging assembly, which includes illumination means, captures images of the target area through the balloon-expanding medium and the balloon material.

US2005/0197530 discloses a catheter having an imaging means and illumination means disposed on a distal end thereof, and a treatment probe having a working lumen that may be distally extended from the distal end of the catheter. Treatment tools such as sensors for monitoring changes in pressure, color, oxygen saturation, flow rate, and echo timing may be passed through the working lumen to sense information from a target area. A balloon is disposed between the distal end of the catheter and a distal end of the treatment probe, such that, when the treatment probe is distally advanced, the balloon extends along the length of the distally exposed treatment probe, and may be inflated with a transparent material. Further, the treatment probe is composed of a transparent material so that the imaging means and the illumination means, which are disposed inside the balloon, can transmit and receive light waves through the balloon, the inflating medium, and the treatment probe to provide an operator with an image of the area being treated.

US 5,178,130 discloses and endoscope system having a first and second electronic endoscopes having illumination systems and camera systems thereon which are synchronized to prevent deterioration in picture quality.

The present invention has been made in order to solve the above-described problem, and has as its object to provide an endoscope and an endoscope system in which positioning of the distal end portion is easy and stable observation is possible even if the distal end portion of the observation probe is thin.

### Disclosure of Invention

### Means for Solving the Problem

These objects are achieved by an endoscope according to claim 1. The endoscope of the present invention has an insertion portion which can be inserted into a subject, an abutment portion which is provided at a distal end portion of the insertion portion and which can contact with the subject, increasing means for increasing the abutment area of the abutment portion, and observation means provided at the distal end portion for observing the subject which is abutted or spaced by a predetermined distance by the abutment portion.

### Brief Description of the Drawings

Fig. 1 is a view showing a whole configuration of an endoscope observation apparatus of a first embodiment of the present invention;
Fig. 2 is an enlarged sectional view of a distal end of an insertion portion of an endoscope in the endoscope observation apparatus of Fig. 1; the view shows a state in which a high magnification observation probe is housed in a forceps channel;
Fig. 3 is an enlarged sectional view including an image pickup unit in a state in which the high magnification observation probe of Fig. 2 is protruded, an abutting balloon abuts a mucous membrane of a living body of an observation region while being expanded, and a region of interest is observed;
Fig. 4 is an enlarged sectional view including an illumination optical system in a state in which the high magnification observation probe of Fig. 2 is protruded, an abutting balloon abuts a mucous membrane of a living body of an observation region while being expanded, and a region of interest is observed;
Fig. 5 is an enlarged sectional view of a normal observation probe, showing a zoom mechanism portion disposed at the distal end of the insertion portion of the endoscope of Fig. 2;
Fig. 6 is a view seen along an arrow A of Fig. 2; the view shows an arrangement of an objective lens window and an illumination lens window and an observation probe observation window in the endoscope insertion portion distal end;
Fig. 7A is a view showing a state in which the high magnification observation probe is still housed in the endoscope insertion portion distal end of Fig. 2;
Fig. 7B is a view showing a state in which the high magnification observation probe protrudes from the endoscope insertion portion distal end;
Fig. 7C is a view showing a state in which the abutting balloon of the distal end portion of the high magnification observation probe has expanded;
Fig. 7D is a view showing a state in which the expanded abutting balloon is about to abut the region of interest of the observation region;

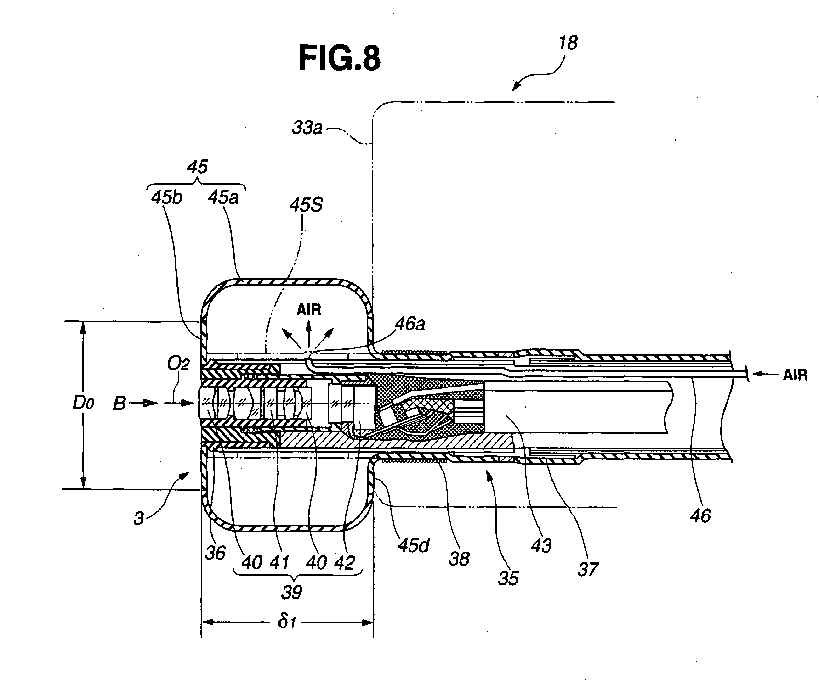

Fig. 8 is a sectional view of the high magnification observation probe of Fig. 2 in the state of Fig. 7D;
Fig. 9 is a view seen along an arrow B of Fig. 8;
Fig. 10 is a view of a variation of the abutting balloon of the high magnification observation probe of Fig. 3 in an expanded state, corresponding to the view seen along the arrow B of Fig. 8;
Fig. 11 is a side view of a variation of the abutting balloon of Fig. 8;
Fig. 12 is a side view showing an expanded state of another variation of the abutting balloon of the high magnification observation probe of Fig. 3;
Fig. 13 is a sectional view of a high magnification observation probe of a second embodiment of the present invention;
Fig. 14 is a view seen along an arrow C of Fig. 13;
Fig. 15 is a sectional view of an observation probe of a high magnification observation probe of a third embodiment of the present invention;
Fig. 16 is a view seen along an arrow D of Fig. 15;
Fig. 17 is a sectional view of a high magnification observation probe of a fourth embodiment of the present invention;
Fig. 18 is a view seen along an arrow E of Fig. 17;
Fig. 19 is a sectional view of a high magnification observation probe of a fifth embodiment of the present invention; and
Fig. 20 is a view seen along an arrow F of Fig. 19.

### Best Mode for Carrying Out the Invention

Embodiments of the present invention will be described in detail below with reference to the drawings.

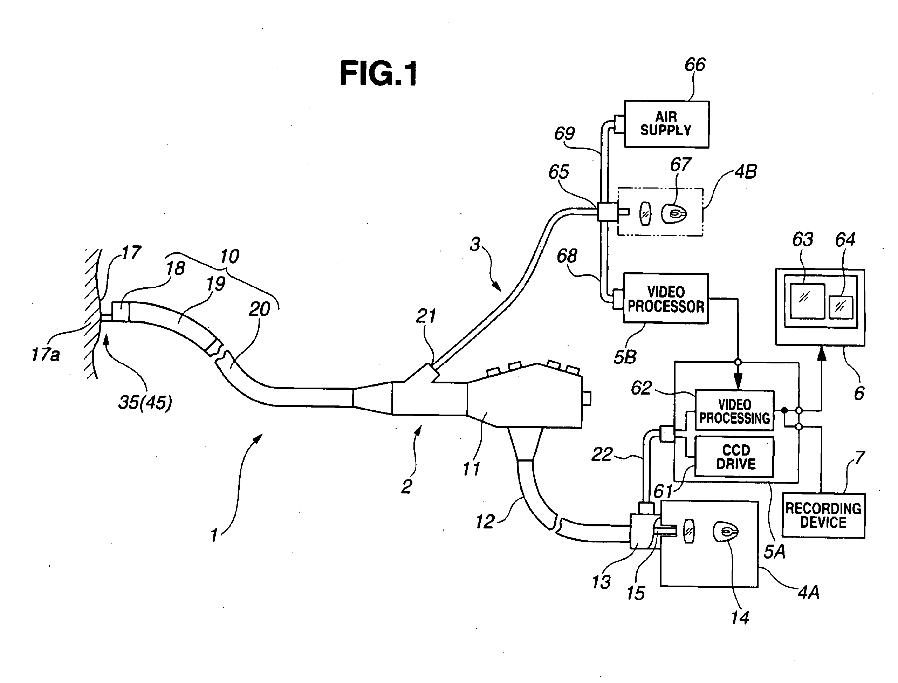

As shown in Figs. 1 to 4, an endoscope observation apparatus 1 of a first embodiment of the present invention has an endoscope 2, a high magnification observation probe 3, a light source device 4A, a video processor 5A, a monitor 6, an air supply device 66, a video processor 5B, and a recording device 7.

The endoscope 2 has an insertion portion 10 which is a first endoscope and which can be inserted into a body cavity which is a subject. The high magnification observation probe 3 is a second endoscope which can perform optical high magnification observation, and is inserted through a forceps channel (also referred to as a treatment instrument channel) 23 of the endoscope 2 so as to be movable back and forth. The high magnification observation probe 3 contains a high magnification image pickup unit 39 which is high magnification observation means. The light source device 4A supplies illumination light to a light guide of the endoscope 2. The video processor 5A performs signal processing for an image pickup unit 27 for normal observation contained in the endoscope 2. The monitor 6 displays video signals outputted from the video processor 5A. The air supply device 66 is fluid supply means for supplying air to an abutting balloon of the high magnification observation probe 3, and is also the increasing means. The video processor 5B performs signal processing for the image pickup unit 39 provided in the high magnification observation probe 3. The recording device 7 records video signals outputted to the monitor 6.

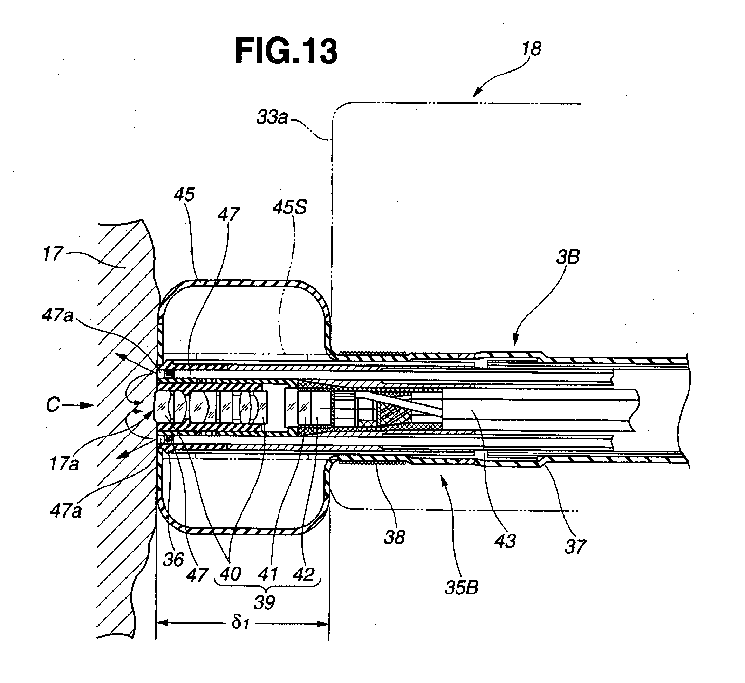

A light source device 4B described in Fig. 1 is a light source device for supplying illumination light to a light guide 47 of a high magnification observation probe 3B (see Fig. 13) in an endoscope observation apparatus of a second embodiment described later. The high magnification observation probe 3 of the present embodiment does not have an incorporated illumination light source, so that the light source device 4B is unnecessary.

The endoscope 2 has an elongated insertion portion 10 having flexibility, an operation portion 11 provided at a rear end of the insertion portion 10, and a universal cord 12 extending out from a side portion of the operation portion 11. The insertion portion 10 contains an image pickup unit 27 which is normal observation means having normal magnification which is variable. A connector 13 provided at a proximal end portion of the universal cord 12 is detachably connected to the light source device 4A.

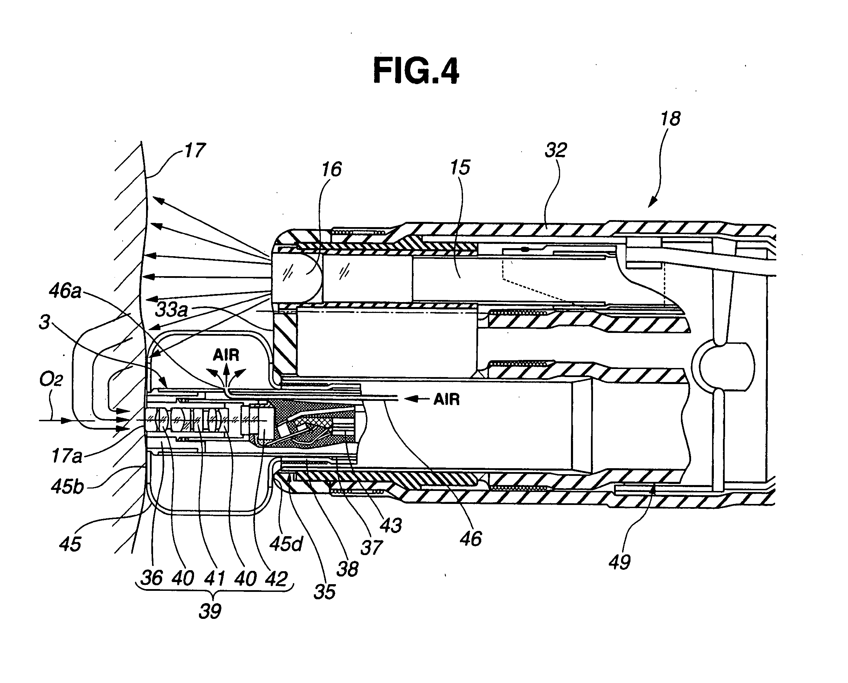

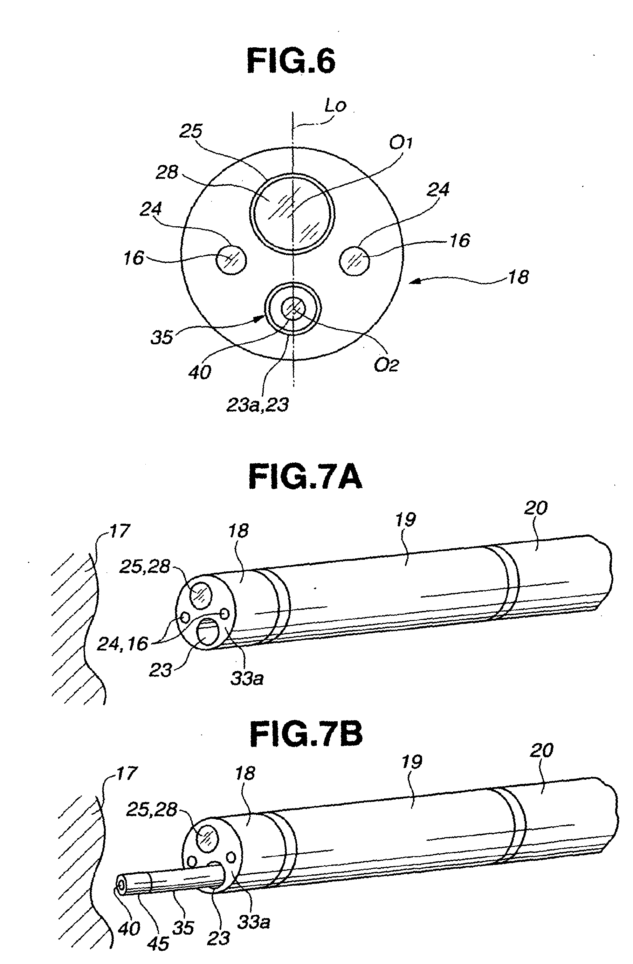

The light source device 4A contains a lamp 14 which generates white light. The white light by the lamp 14 is collected by a lens and enters a light guide 15 of a light guide base portion protruding from the connector 13. This white light is transmitted by the light guide 15 and exits from the distal end face of the insertion portion 10 through an illumination lens 16 (see Figs. 4, 6) to illuminate an observation region 17 such as a diseased part.

The insertion portion 10 comprises a rigid distal end portion 18, a freely bendable bending portion 19 provided at the rear end of the distal end portion 18, and a long flexible portion 20 extending from the rear end of the bending portion 19 to the front end of the operation portion 11. The bending portion 19 can be bent in any direction among vertical and lateral ones by operation of an unshown bending knob provided in the operation portion 11.

An illumination window 24 and an observation window (image pickup window) 25 are provided in a distal end portion main body 26 which configures the distal end portion 18 of the insertion portion 10. The distal end portion of the light guide 15 and the illumination lens 16 and the like at the distal end face of the light guide 15, which configure an illumination optical system, are arranged in the illumination window 24. The observation window (image pickup window) 25 is provided adjacent to the illumination window 24. In the observation window 25, an objective lens system 28 for zoom type normal observation having an observation optical axis O1 is disposed in a state of being retained by a lens frame. A zoom lens 28a is contained in the objective lens system 28.

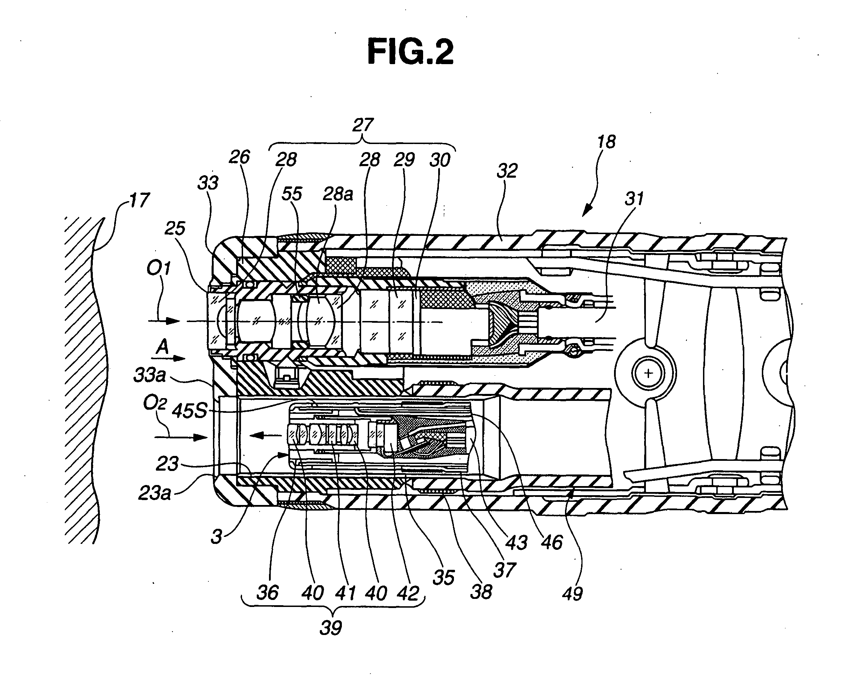

A CCD 30 which is a charge-coupled device, for example, is arranged behind the objective lens system 28 at the position of image formation as a solid image pickup device, thereby configuring an image pickup unit 27 (Fig. 2). The CCD 30 is image pickup means for normal observation, which subjects an optical image formed to photoelectric conversion. A protective glass 29 and an optical filter are disposed in front of the CCD 30.

A treatment instrument insertion aperture 21 is provided near the front end of the operation portion 11, where a treatment instrument, high magnification observation probe 3 or the like can be inserted. The treatment instrument insertion aperture 21 communicates therein with the forceps channel 23 (see Fig. 2), part of which is configured by a flexible tube 49 provided along the longitudinal direction of the insertion portion 10.

In the distal end portion main body 26 is formed a channel hole portion communicating with the flexible tube 49 which forms the forceps channel 23. A distal end portion 35 of the high magnification observation probe 3 inserted in the forceps channel 23 can freely appear and disappear from a distal end opening portion 23a of the forceps channel 23. The distal end portion 35 of the high magnification observation probe 3 contains a high magnification image pickup unit 39. The observation optical axis of the observation window of the distal end portion 35 is denoted by optical axis 02.

A bending piece configuring the most distal end of the bending portion 19 is fixedly attached to the rear end of the distal end portion main body 26. The outside of the bending piece is water-tightly covered by an exterior member 32 comprising a rubber tube or the like having good bendability.

A front end face 33a of a distal end cover 33 of the distal end portion 18 of the insertion portion 10 is formed with a plane perpendicular to the optical axis O1. The observation window 25, the illumination window 24 and the distal end opening portion 23a of the forceps channel 23 are disposed on the front end face 33a. In addition, as shown in Fig. 6, the optical axis 02 of the observation window 25 as well as the 02 of the observation window of the distal end portion 35 on the distal end opening portion 23a of the forceps channel 23 are arranged on a straight line L0. The two illumination windows 24 are disposed across the straight line L0 opposite to each other. Therefore, illumination light can efficiently illuminate the photographic subjects both of the image pickup unit 27 and the image pickup unit 39.

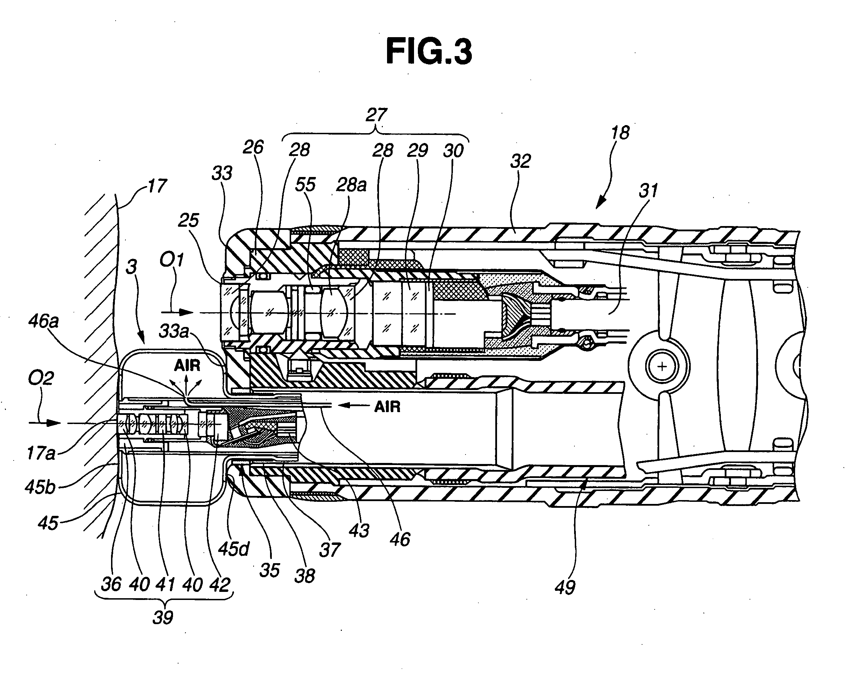

When high magnification observation is performed, the distal end portion 35 of the high magnification observation probe 3 is protruded through the distal end opening 23a of the forceps channel 23 as shown in Figs. 1, 3. At this time, a front end face 45b of an abutting balloon 45 in an expanded state descried later abuts the surface of a region of interest 17a which is desired to be observed locally at high magnification in the observation region 17 which is a subject such as a mucous membrane of a living body. Then, the distal end portion 35 is fixed in a predetermined position in the abutment state so that high magnification observation of the histological microstructure of the region of interest 17a can be performed through the observation window of the distal end portion 35.

The detailed structure around the distal end portion 35 of the high magnification observation probe 3 will be described later using Figs. 2 to 4.

The distal end of a signal cable 31 is connected to the CCD 30 of the image pickup unit 27 shown in Fig. 2. The rear end side of the signal cable 31 is connected to a connector receptacle at the side portion of the connector 13, and is detachably connected to the video processor 5A through a signal cable 22 connected to the connector receptacle.

The video processor 5A contains a CCD drive circuit 61 and a video processing circuit 62. The CCD drive circuit 61 generates a CCD drive signal for driving the CCD 30. The video processing circuit 62 performs signal processing of an image pickup signal outputted from the CCD 30 by application of the CCD drive signal, and generates a video signal.

The video signal generated by the video processing circuit 62 is outputted to the monitor 6, and is displayed in a normal observation image display area 63 of the monitor 6 as a normal observation endoscope image.

The distal end of a signal cable 43 is connected to a CCD 42 in the high magnification image pickup unit 39 side. The rear end side of the signal cable 43 is detachably connected to the video processor 5B through a signal cable 68 extending out from a connector portion 65, for example.

The video processor 5B is configured to contain a CCD drive circuit and a video processing circuit, similarly to the video processor 5A. The video signal outputted from the video processor 5B and corresponding to an image pickup signal of image pickup by the CCD 42 is inputted to the video processing circuit 62 of the video processor 5A.

The video processing circuit of the video processors 5A, 5B performs signal processing to generate an RGB color signal, for example, which is a video signal corresponding to an image pickup signal of the CCD 42 under illumination of white light, and outputs the generated video signal to the video processing circuit 62.

In addition to a normal observation video signal, a high magnification observation video signal outputted from the video processor 5B is inputted to the video processor 5A and, through an unshown mixer therein, they are outputted to the monitor 6. Then, a high magnification (enlarged) observation image by the high magnification observation probe 3 is displayed in a high magnification observation image display area 64 adjacent to the endoscope normal observation image display area 63 of the monitor 6.

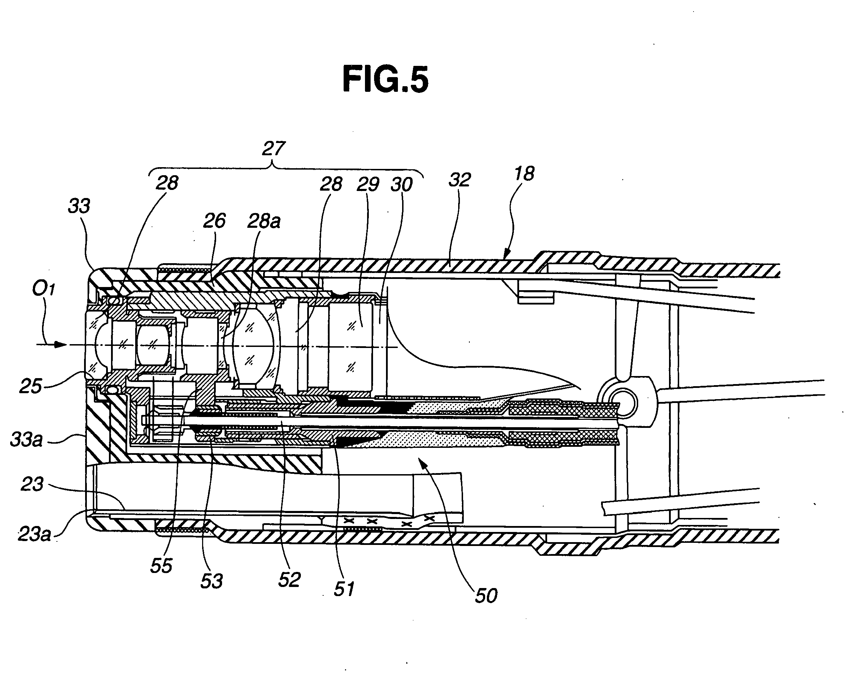

The image pickup unit 27 contained in the endoscope 2 has variable magnification as described above, and a zoom drive mechanism 50 is disposed laterally to the image pickup unit 27 as shown in Fig. 5. The zoom drive mechanism 50 performs zooming by driving the zoom lens 28a of the objective lens system 28 back and forth.

The zoom drive mechanism 50 is configured to have a wire 52, a support member 51, a lens frame 55 and a coupling member 53. The wire 52 is disposed parallel to the observation optical axis O1. The support member 51 is configured with a tube member or the like and supports the wire 52 so that the wire is movable back and forth. The lens frame 55 retains the zoom lens 28a. The coupling member 53 is fixed to the lens frame 55 by screwing and fixed to the wire 52 by bonding.

When zooming of a normal observation image by the image pickup unit 30 is performed, a zoom operation dial (not shown) provided in the operation portion 11 is rotatively operated. Then, with the rotative operation the wire 52 moves back and forth, so that the zoom lens 28a moves back and forth along the observation optical axis O1 by means of the coupling member 53 thereby to perform zooming.

The distal end portion 35 of the high magnification observation probe 3 is formed with a rigid and light-blocking thin cylindrical body 36 shown in Figs. 2, 3. The distal end of a flexible sheath (flexible tube) 37 is water-tightly fixed to the rear end of the cylindrical body 36, thereby forming a flexible insertion portion which can be inserted through the forceps channel 23.

The high magnification image pickup unit 39 which is observation means enabling high magnification observation is disposed in the hollow portion of the cylindrical body 36. The high magnification image pickup unit 39 is configured by a high magnification objective lens system 40 attached to a lens frame provided in the central portion in the cylindrical body 36, an optical filter 41, and a CCD 42 which is a solid image pickup device fixed at the position of image formation behind the lens system 40. As for the observation magnification of the high magnification image pickup unit 39, the monitor magnification is in the order of 200 to 1000 times, for example, and tissue cells, ducts and the like can be observed. The observation range is 700 µm x 700 µm or less and the observation resolving power is 5 µm or less.

An abutting balloon 45, having air-tightness and comprising a stretchable and expandable membrane member in the form of a bag, is mounted to the outer peripheral portion of the cylindrical body 36. The root portion of the abutting balloon 45 is sealed on the cylindrical body 36 by winding with a thread winding portion 38, while the rear end portion is fixed to the flexible sheath 37 by bonding. An air supply hole 46a of an air supply tube 46, which is increasing means disposed inserted in the inner periphery of the observation probe 3, is positioned in the inner periphery of the abutting balloon 45. Air supplied from the air supply device 66 can be supplied through a coupling tube 69 to the air supply tube 46. The balloon 45 becomes the portion abutting the subject in an expanded state.

In a normal observation state, the distal end portion 35 of the observation probe 3 is not protruding from the front end face 33a of the distal end portion 18 of the insertion portion 10. In addition, the abutting balloon 45 is in a contracted state 45S as shown in Fig. 2 and is retained in the forceps channel with some clearance, adhering to the outer periphery of the cylindrical body 36. When high magnification observation is performed, the distal end portion 35 is protruded out of the forceps channel 23 and thereafter air is supplied to the abutting balloon 45 to expand the abutting balloon 45S which has been in a contracted state.

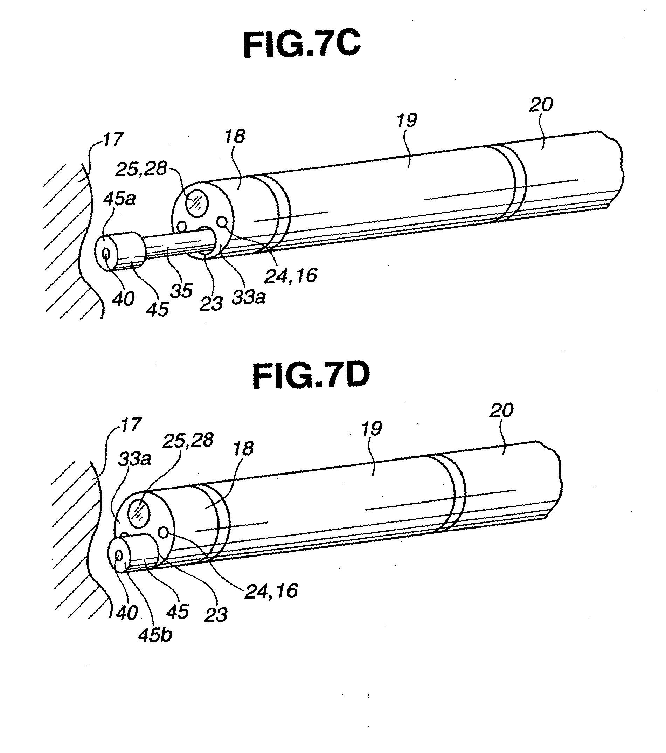

Specifically, when high magnification observation is performed, an operator operates the observation probe 3 arranged in the forceps channel 23 of the insertion portion 10 of the endoscope 2 to cause it to move forward. Then, as shown in Fig. 7B, the distal end portion 35 is protruded forward from the front end face 33a of the distal end cover 33. Thereafter, air is supplied from the air supply device 66 to expand the abutting balloon 45 as shown in Fig. 7C.

After the abutting balloon 45 has been expanded, the observation probe 3 is slightly withdrawn. Then, a rear face portion 45d of the abutting balloon 45 in an expanded state abuts the front end face 33a of the distal end portion 18, as shown in Figs. 7D, 3. In this state, the front face portion 45b which is the abutting face of the abutting balloon 45 is caused to abut a portion around the region of interest 17a of the observation region 17. Then, the observation window portion on a front face of the objective lens system 40 of the image pickup unit 39 is in close contact with the region of interest 17a, enabling high magnification observation by the image pickup unit 39.

A length δ1 between the front face portion 45b and the rear face portion 45d of the abutting balloon 45 in an expanded state is set to be the same distance as the near point observation side depth of field of the image pickup unit 27 of the endoscope 2 side. Therefore, in a zoom magnification state of the endoscope 2, the distance between the subject and the observation window can be stably maintained even when the depth of field is shallow. Observation can be easily performed even in a zoom state of the normal observation optical system.

When the abutting balloon 45 is in an expanded state, desirably the front face portion 45b and the rear face portion 45d have a shape close to a plane, and particularly such a shape that a large area of the front face portion 45b abuts the observation region 17. Further, an outer peripheral portion 45a desirably has a shape close to a cylindrical shape. In addition, the front face portion 45b is desirably a non-slippery surface.

If the front face portion 45b of the abutting balloon 45 in an expanded state has a shape close to a plane as described above, when the distal end portion 35 of the observation probe 3 abuts the region of interest 17a, the local load applied to the abutment region can be alleviated by increasing the abutment area by means of the front face portion 45b. Further, if the front face portion 45b is formed of a non-slippery material as described later, displacement of the distal end portion 35 in an observation state can be prevented.

In addition, when normal observation needs to be performed additionally in a state in which high magnification observation is possible, the vignetting of the field of view of the objective lens system 28 of the endoscope 2 side will be reduced with the outer peripheral portion 45a of the abutting balloon 45 having a cylindrical shape.

As for illumination light at the time of high magnification observation by the distal end portion 35 of the observation probe 3, illumination light projected from the illumination lens 16 for illuminating the field of view of the objective lens system 28 gets in the interior of the region of interest 17a and is reflected as shown in Fig. 4. The image of the region of interest 17a is taken in through the high magnification objective lens system 40 of the distal end portion 35 of the observation probe 3. Therefore, the abutting balloon 45 is preferably formed of a transparent material or a semi-transparent material so that illumination light from the illumination window 24 of the endoscope 2 side may reach the region of interest 17a.

By adopting a transparent material or a semi-transparent material for the abutting balloon 45 as described above, observation is enabled without providing a light guide 47 in the high magnification observation probe 3B as shown in Fig. 13. Therefore, a high magnification observation probe of a small diameter can be obtained, so that application to a wide range of inspections is enabled.

In order to obtain an abutting balloon 45 which has a cylindrical shape when in an expanded state as described above, it is preferable that in the area of a diameter **D0** of the front face portion 45b, for example, of the abutting balloon 45, a non-slippery material less stretchable than other portions is disposed by two shot molding, or the thickness is great. Similarly, it is preferable that also in the rear face portion 45d side, a less stretchable material is disposed, or the thickness is great. In addition, minute concavity and convexity may be provided in the front face portion 45b in order to render it non-slippery as described above. In addition, it is preferable that also in the middle portion of the outer periphery of the cylindrical portion along the circumferential direction, a material less stretchable than other portions is disposed by two shot molding, or the thickness is great.

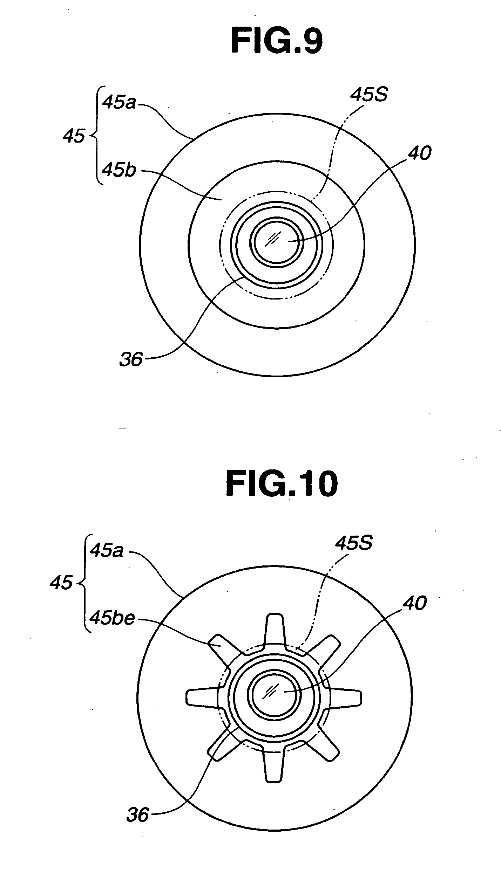

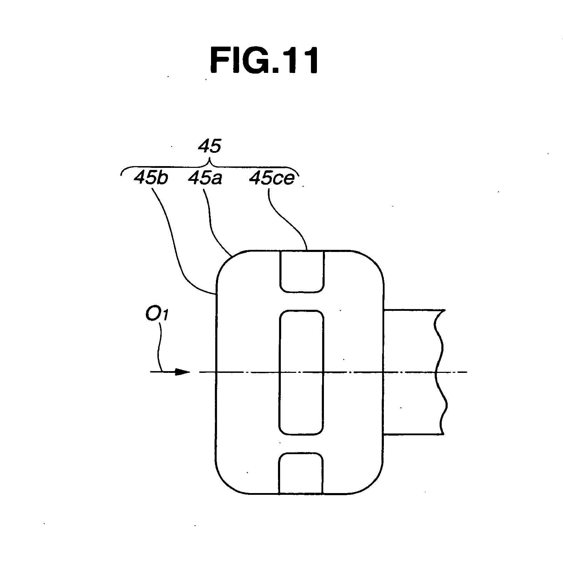

Further, as in an abutting balloon of a variation shown in Fig. 10, less stretchable portions 45be extending in the radial direction may be provided by two shot molding in the circumferential direction of the front face portion 45b. In addition, in order to maintain the cylindrical shape of the abutting balloon in the expanded state, less stretchable portions 45ce may be disposed along the outer peripheral portion of the abutting balloon 45 as shown in a variation of Fig. 11.

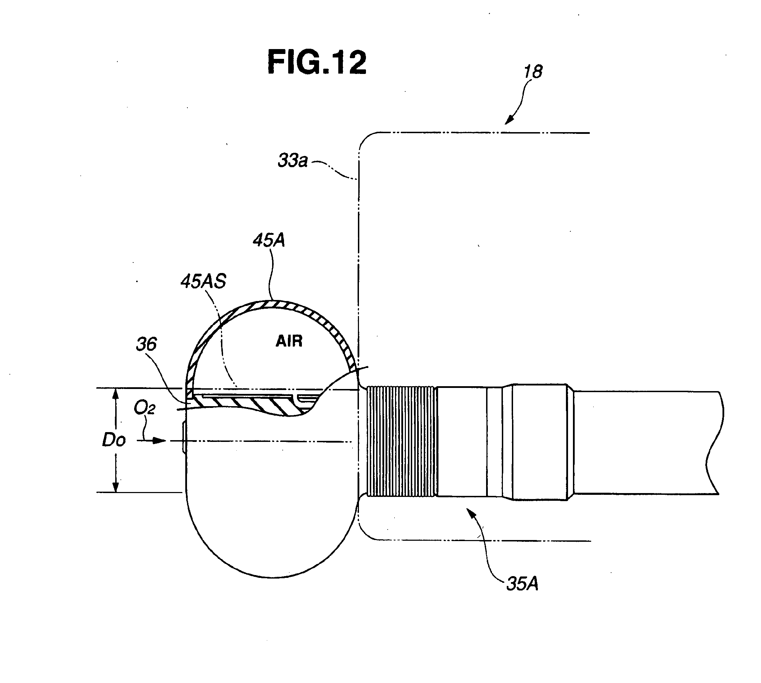

Further, an abutting balloon 45A as shown in a side view of Fig. 12 is also contemplated as a variation of an abutting balloon for which care is not taken that the abutting balloon in the expanded state has its front face portion 45b in the form of a plane and its outer diameter in the form of a cylinder.

Here, observation operation by the endoscope observation apparatus 1 of the present embodiment having the above-described configuration will be described.

When normal observation and high magnification observation of an observation region 17 of a mucous membrane of a living body are performed, the endoscope 2 is inserted in a body cavity, and the observation region 17 of a mucous membrane or the like is observed by the normal observation image pickup unit 27 provided in the distal end portion 18 of the endoscope 2. Then, if there exists a region of interest 17a with respect to which observation of the histological microstructure is desired, treatment of staining the region of interest 17a is performed; thereafter, a tube (not shown) which is pigment dispersing means is withdrawn from the insertion aperture 21; subsequently, the observation probe 3 is inserted in the forceps channel 23 from the insertion aperture 21 as shown in Fig. 1. Then, the distal end portion 35 of the observation probe 3 is protruded from the distal end opening 23a of the forceps channel 23 and thereafter the contracted abutting balloon 45AS is expanded as described above, and in a state of normal observation by the endoscope 2, the front face portion 45b of the abutting balloon 45A is pushed onto (brought into contact) the area around the region of interest 17a as shown in Fig. 3 formerly.

Positioning can be performed without displacement by bringing the observation window 28 provided in the distal end face of the high magnification observation probe 3 located in the middle of the abutting balloon 45 into close contact with the surface of the region of interest 17a as described above with the region of interest 17a being in the range of observation by the endoscope 2. That is, an observation state as shown in Fig. 3 can readily be set.

In a high magnification observation state, illumination light from the light source device 4A is projected from the illumination lens 16. The illumination light intrudes into the inner side of the mucous membrane surface with which the distal end face of the observation probe 3 is brought into contact, and is scattered by tissue and the like therein to illuminate the region of interest 17a. An optical image of the region of interest 17a is formed on the CCD 42 arranged at the position of image formation of the high magnification objective lens system 40, the distal end face of which is pushed onto the region.

The front face portion 45b of the abutting balloon 45 abuts the portion around the region of interest 17a, and the observation window 28 of the distal end portion 35 of the observation probe 3 is pushed onto the region of interest 17a. Therefore, the distal end portion 35 of the observation probe 3 is prevented from being unstable and changing its position. That is, an optical image focused on the region of interest 17a near the end face of the observation window can be formed on the CCD 42 with the observation probe 3 being held without displacement.

An image formed on the CCD 42 is subjected to photoelectric conversion by the CCD 42 and is converted to a video signal by the video processing circuit in the video processor 5B, and a high magnification observation image is displayed adjacently to the endoscope image in the monitor 6. Therefore, it is easy for an operator to accurately diagnose the region of interest 17a by observing a high magnification observation image.

As described above, according to the endoscope observation apparatus 1 of the present embodiment, since the front face portion 45b of the abutting balloon 45 in the expanded state disposed in the distal end of the high magnification observation probe 3 abuts the portion around the region of interest 17a at the time of high magnification observation, backlash due to clearance between the forceps channel 23 and the observation probe 3 is absorbed, so that the observation window of the distal end portion 35 can be held without displacement with respect to the region of interest 17a.

In addition, by the front face portion 45b of the abutting balloon 45 reliably abutting the portion around the region of interest 17a, an optical image focused on the region of interest 17a near the observation window on a front face of the objective lens system 40 can be formed on the CCD 42. In addition, the wider front face portion 45b of the abutting balloon 45 abuts the portion around the region of interest 17a. Therefore, even if the distal end portion 35 of the high magnification observation probe 3 is of a small diameter, the distal end portion 35 can be prevented from being buried in the region of interest 17a. Further, since the length δ1 of the abutting balloon 45 corresponds to the near point side observation distance (depth of field) of the endoscope 2 where clear observation is possible, observation can be performed smoothly without the need to change the distance between the distal end portion of the endoscope 2 and living body tissue at the time of switching from a high magnification observation state to observation by the endoscope 2.

In addition, any normal endoscope having a forceps channel can reliably perform high magnification (magnifying) observation by applying the high magnification observation probe 3 according to the present embodiment.

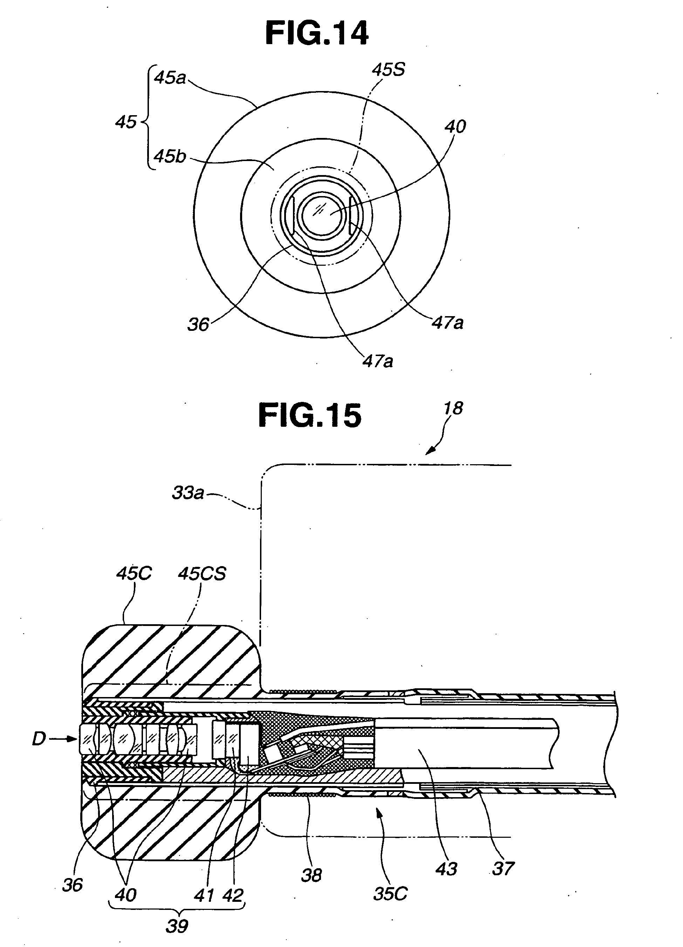

Next, a high magnification observation probe of a second embodiment which is applied to the endoscope observation apparatus of the present invention will be described with reference to Figs. 13, 14.

A high magnification observation probe 3B which is a second endoscope of the present embodiment contains a light guide 47 which is illumination means around the image pickup unit 39 in the probe as shown in Fig. 13. As for the configuration in other respects, a high magnification image pickup unit 39 is contained in the distal end portion 35B and an expandable abutting balloon 45 comprising a stretchable member is mounted on the outer periphery of the cylindrical body 36 of the distal end portion similarly to the high magnification observation probe 3 applied to the first embodiment. In the following, portions different from the high magnification observation probe 3 will be described.

The high magnification observation probe 3B has a light guide base protruding from the connector portion 65 as shown in Fig. 1, and the light guide base is detachably connected to the light source device 4B.

White light by a lamp 67 in the light source device 4B enters the light guide 47 from the light guide base through a lens. In a high magnification observation state by the high magnification observation probe 3B, illumination light is transmitted by the light guide 47 to the distal end of the distal end portion 35B. The illumination light is projected from an illumination window 47a disposed around the objective lens 40. At this time, since the abutting balloon 45 abuts the portion near the region of interest 17a and the distal end face of the distal end portion is in contact with the region of interest 17a, illumination light is radiated toward the inner side of the mucous membrane surface and scattered by tissue and the like therein. An image of the tissue illuminated by the illumination light is taken in by the high magnification objective lens system 40 and formed on the image formation surface of the CCD 42. The optical image of the region of interest 17a is subjected to photoelectrical conversion into an electrical signal by the CCD 42, and the electrical signal is formed into a video signal by the video processor 5B.

According to an endoscope observation apparatus to which the high magnification observation probe 3B of the present embodiment is applied, in addition to the formerly described effects by the first embodiment, the area whose image is formed on the CCD 42 is illuminated by means of the light guide 47 provided around the high magnification objective lens system 40 independent of the endoscope 2, so that the region of interest 17a can be properly illuminated at the time of high magnification observation.

In addition, in the present embodiment, since the endoscope 2 and the high magnification observation probe 3B respectively have an independent illumination means provided, observation can be performed with sufficient amounts of light in both normal observation and high magnification observation.

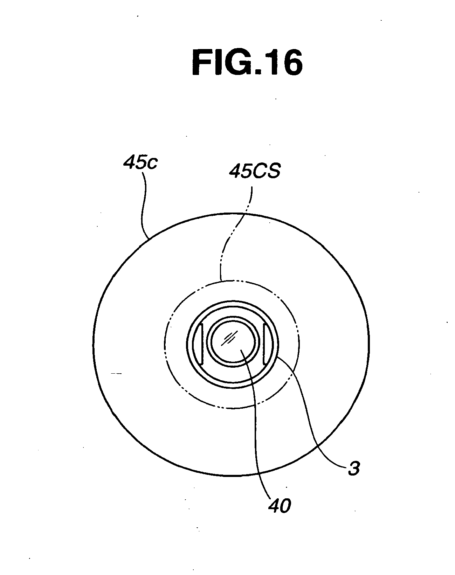

Next, a high magnification observation probe of a third embodiment which is applied to an endoscope observation apparatus of the present invention will be described with reference to Figs. 15, 16.

As shown in Fig. 15, in a high magnification observation probe 3C which is a second endoscope of the present embodiment, the material of the abutting balloon mounted on the outer periphery of the cylindrical body 36 configuring the high magnification observation probe 3 in the first embodiment is different. Specifically, an abutting balloon 45C mounted to the cylindrical body 36 of the high magnification observation probe 3C of the present embodiment is not a membrane member in the form of a bag, but a cylindrical member which can be expanded and compressed, such as one made of foamed synthetic rubber, for example. Therefore, in the present embodiment, a supply device for supplying air which expands the abutting balloon 45C is unnecessary.

As for the configuration in other respects, a high magnification image pickup unit 39 is contained in the distal end portion 35C similarly to the high magnification observation probe 3 applied to the first embodiment. In the following, portions different from the high magnification observation probe 3 will be described.

The abutting balloon 45C applied to the high magnification observation probe 3C of the present embodiment is housed in a state (45CS) of being compressed by the inner periphery of the forceps channel 23 of the endoscope 2 when being inserted in the channel. In a state in which the distal end portion 35C protrudes outwardly of the channel for high magnification observation, the abutting balloon 45C is in an expanded state as shown in Fig. 15.

That is, at the time of high magnification observation, the front face portion of the abutting balloon 45C in the expanded state abuts the portion around the region of interest 17a of the observation region 17 and observation by the high magnification image pickup unit 39 can be performed in the same way as in the case of the high magnification observation probe 3 applied to the first embodiment.

As described above, when the high magnification observation probe 3C of the present embodiment is applied, in addition to the formerly described effects by the first embodiment, the air supply device 66 is unnecessary, so that the system configuration is simple. In addition, the shape of the abutting balloon 45C in the expanded state is stable and it can abut the observation region 17 reliably.

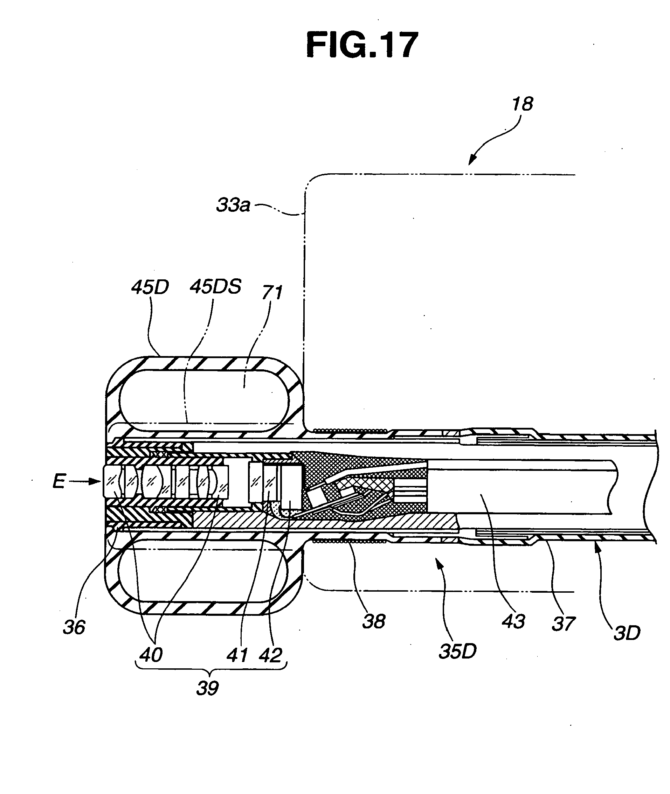

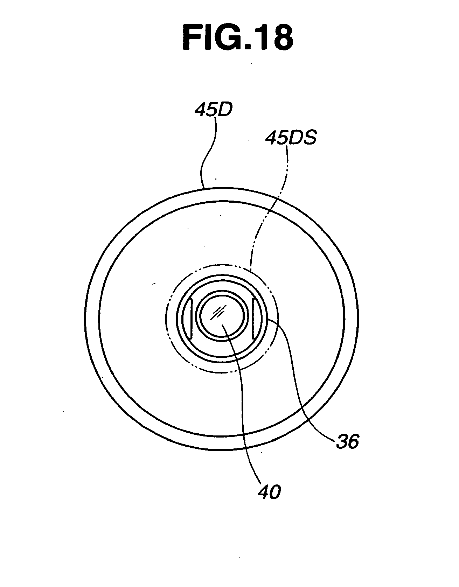

Next, a high magnification observation probe of a fourth embodiment which is applied to an endoscope observation apparatus of the present invention will be described with reference to Figs. 17, 18.

As shown in Fig. 17, in a high magnification observation probe 3D which is a second endoscope of the present embodiment, the shape of the abutting balloon mounted to the outer periphery of the cylindrical body 36 configuring the high magnification observation probe 3 in the first embodiment is different. Specifically, an abutting balloon 45D mounted to the outer periphery of the cylindrical body 36 of the high magnification observation probe 3D of the present embodiment is formed with synthetic rubber or the like which can be expanded and compressed, having an air chamber 71. In the abutting balloon 45D, a supply device for supplying air for expansion of the abutting balloon is unnecessary. As for the configuration in other respects, a high magnification image pickup unit 39 is contained in the distal end portion 35D similarly to the high magnification observation probe 3 applied to the first embodiment. In the following, portions different from the high magnification observation probe 3 will be described.

When in a state of being inserted in the forceps channel 23 of the endoscope 2, the abutting balloon 45D applied to the high magnification observation probe 3D of the present embodiment is housed in a state (45DS) in which the air chamber 71 is compressed by the inner periphery of the channel. In a state in which the distal end portion 35D protrudes outwardly of the channel for high magnification observation, the abutting balloon 45D is in a state of being expanded in the form of a cylinder, with the air chamber 71 being expanded, as shown in Fig. 17.

That is, at the time of high magnification observation, the front face portion of the abutting balloon 45D in the expanded state abuts the portion around the region of interest 17a of the observation region 17, and observation by the high magnification image pickup unit 39 can be performed in the same way as in the case of the high magnification observation probe 3 applied to the first embodiment.

As described above, when the high magnification observation probe 3D of the present embodiment is applied, in addition to the formerly described effects by the first embodiment, the air supply device 66 is unnecessary, so that the system configuration is simple. In addition, the shape of the abutting balloon 45D in the expanded state is stable and it can abut the observation region 17 reliably.

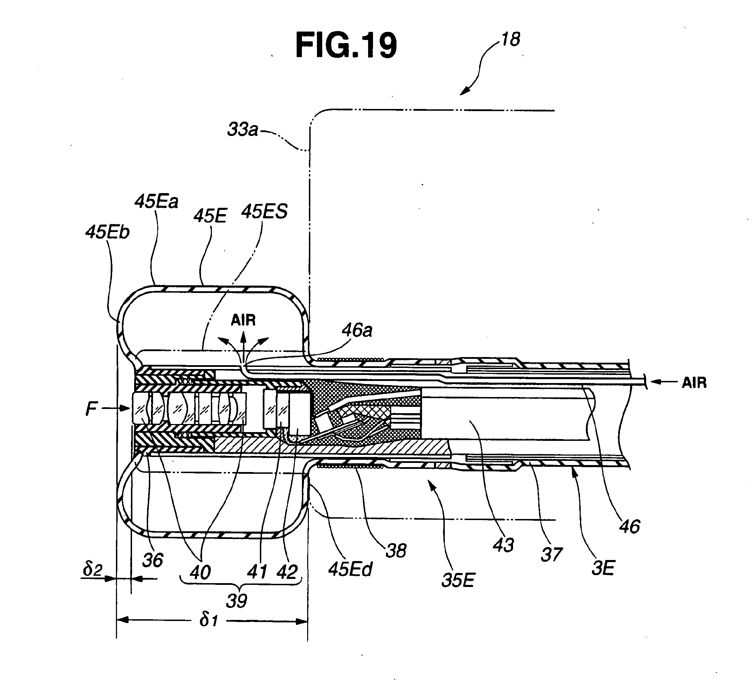

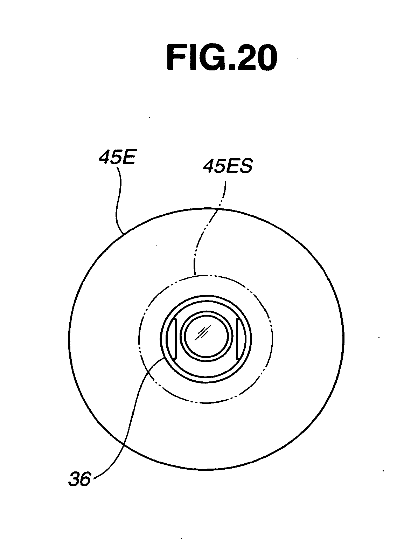

Next, a high magnification observation probe of a fifth embodiment which is applied to an endoscope observation apparatus of the present invention will be described with reference to Figs. 19, 20.

As shown in Fig. 19, in a high magnification observation probe 3E which is a second endoscope of the present embodiment, the shape of the abutting balloon mounted to the outer periphery of the cylindrical body 36 configuring the high magnification observation probe 3 in the first embodiment is different. Specifically, while an abutting balloon 45E mounted to the outer periphery of the cylindrical body 36 of the high magnification observation probe 3E of the present embodiment is a stretchable and expandable membrane member in the form of a bag similarly to the abutting balloon 45, the shape of the front face portion of the abutting balloon 45E in the expanded state is different. As for the configuration in other respects, a high magnification image pickup unit 39 is contained in the distal end portion 35E similarly to the high magnification observation probe 3 applied to the first embodiment. In the following, portions different from the high magnification observation probe 3 will be described.

When in a state of being inserted in the forceps channel 23 of the endoscope 2, the abutting balloon 45E applied to the high magnification observation probe 3E of the present embodiment is not supplied with air and is retained in a contracted state (45ES) in the form of being in close contact with the outer peripheral portion of the cylindrical body 36 of the distal end portion 35E. After the distal end portion 35E has been made to protrude outwardly of the channel for high magnification observation, air supplied from the air supply device 66 enters into the abutting balloon 45E from the air supply hole 46a, and the abutting balloon 45E expands so as to be in a state of the balloon being expanded, shown in Fig. 19.

At this time, the front face side of the expanded-state abutting balloon 45E protrudes with a balloon front portion 45Eb bulging by a predetermined length δ2 as compared with the observation window on the front face of the objective lens system 40.

When high magnification observation is performed, the balloon front portion 45Eb of the abutting balloon 45E abuts the region of interest 17a of the observation region 17. In that abutment state, the region of interest 17a and the observation window on the front face of the objective lens system 40 are spaced by the predetermined length δ2. However, the length δ2 is set to be a distance within the depth of field (observable depth) of the high magnification image pickup unit 39 at the time of observation. Therefore, a focused state is obtained in the abutment state of the abutting balloon 45E with the region of interest 17a.

In addition, the protruding balloon front portion 45Eb is formed of a non-slippery material and is non-slippery with respect to the observation region 17. Therefore, the distal end portion 35E of the high magnification observation probe 3E can be held more reliably without displacement.

In addition, the distance δ1 between the end of the balloon front portion 45Eb and the rear end face 45Ed of the abutting balloon 45E is set in accordance with the near point side depth of field (observation depth) of the normal observation image pickup unit 27 of the endoscope 2 side in the same way as in the case of the high magnification observation probe 3. Therefore, at the time of switching from a high magnification observation state by the high magnification observation probe 3 to observation by the endoscope 2, observation can be performed smoothly without the need to change the distance between the distal end portion of the endoscope 2 and the living body tissue.

As described above, when the high magnification observation probe 3E of the present embodiment is applied, in addition to the formerly described effects by the first embodiment, the distal end portion 35E can be held more reliably without displacement in a state in which the abutting balloon 45E in the expanded state abuts the observation region 17.

In addition, since the region of interest 17a and the observation window on the front face of the objective lens system 40 can always be spaced by the predetermined distance [delta]2 at the time of high magnification observation, stable high magnification observation images can be obtained.

## Claims

1. An endoscope system (1), comprising:
a first endoscope (2) comprising:
a first insertion potion (10) adapted to be inserted into a subject;
a channel (23) having an opening (23a) at a distal end portion (18) of the first insertion portion (10);
an observation window (25) at the distal end portion (18) of the first insertion portion (10);
an illumination window (24) adapted for illuminating an observation region at the distal end portion (18) of the first insertion portion (10); and
an image pickup unit (27) adapted for performing normal observation of the observation region (17a);
a second endoscope (3, 3B-D) having a second insertion portion (37) adapted to be inserted into and removed from the channel (23), the second insertion portion (37) comprising a distal end portion (35) adapted to be protruded through the opening (23a);
an abutting balloon (45, 45A, 45C, 45D) provided on an outer peripheral portion of the distal end portion (35) of the second insertion portion (37) and adapted to be expanded and contracted; and
**characterized in that**
the second endoscope (3, 3B-D) further comprises an image pickup unit (39) provided in the distal end portion (18) and adapted to perform high magnification observation of a histological microstructure of a region of interest within the observation region (17a);
wherein, when the abutting balloon (45, 45A, 45C, 45D) is in an expanded state:
a front face portion (45b) of the abutting balloon (45) is adapted to abut around the region of interest (17a);
a rear face portion (45d) of the abutting balloon is adapted to abut a front end face (33a) of the distal end portion (18) of the first insertion portion (10); and
an observation window located at a front face of the image pickup unit (39) for high magnification observation is in close contact with the region of interest (17a).

2. The endoscope system according to claim 1, wherein the abutting balloon (45) is comprised of a transparent or semitransparent material.

3. The endoscope system of claim 1, wherein the abutting balloon (45C) is a cylindrical member (36) made of an expandable and compressible foamed rubber.

4. The endoscope system of claim 1, wherein the abutting balloon (45D) is formed with an expandable and compressible synthetic rubber having a plurality of air chambers (71).

5. The endoscope system according to one of claims 1 to 4, wherein, when the balloon (45, 45A, 45C, 45D) is in the expanded state, the front face portion (45b) and the rear face portion (45d) each have a shape that is generally planar.

6. The endoscope system of claim 5, wherein the front face portion (45b) and the rear face portion (45d) are formed of a non-slippery member.

7. The endoscope system of claim 5, wherein the front face portion (45b) is provided with concavity and convexity.

8. The endoscope system according to claim 6, wherein the front face portion (45b) and the rear face portion (45d) of the abutting balloon are formed to have the generally planar shape in the expanded state by making a thickness of the front and rear face portions (45a, 45d) greater than thicknesses of other portions of the abutting balloon or by disposing a material less stretchable than other portions of the abutting balloon at the front and rear face portions (45a, 45d).

9. The endoscope system according to one of claims 1-8, wherein a position of a surface of the abutting balloon (45) abutting on the region of interest (17a) is provided in a range of a depth of field of the first endoscope (2).

## Patentansprüche

1. Endoskopsystem (1), aufweisend:
ein erstes Endoskop (2), aufweisend:
einen ersten Einführabschnitt (10), der dazu eingerichtet ist, in eine Person eingeführt zu werden;
einen Kanal (23) mit einer Öffnung (23a) am distalen Endabschnitt (18) des ersten Einführabschnitts (10);
ein Beobachtungsfenster (25) am distalen Endabschnitt (18) des ersten Einführabschnitts (10);
ein Beleuchtungsfenster (24), das zum Beleuchten einer Beobachtungszone am distalen Endabschnitt (18) des ersten Einführabschnitts (10) eingerichtet ist; und
eine Bildaufnahmeeinheit (27), die zum Ausführen der normalen Beobachtung der Beobachtungszone (17a) eingerichtet ist;
ein zweites Endoskop (3, 3B-D) mit einem zweiten Einführabschnitt (37), der dazu eingerichtet ist, in den Kanal (23) eingeführt und aus diesem entfernt zu werden,
wobei der zweite Einführabschnitt (37) einen distalen Endabschnitt (35) aufweist, der dazu eingerichtet ist, durch die Öffnung (23a) herauszuragen;
einen anliegenden Ballon (45, 45A, 45C, 45D), der an einem Außenumfangsabschnitt des distalen Endabschnitts (35) des zweiten Einführabschnitts (37) vorgesehen und
dazu eingerichtet ist, sich auszudehnen und zusammenzuziehen; und
**dadurch gekennzeichnet, dass**
das zweite Endoskop (3, 3B-D) ferner eine Bildaufnahmeeinheit (39) im distalen Endabschnitt (18) aufweist, die zur Beobachtung einer histologischen Feinstruktur einer interessierenden Zone innerhalb der Beobachtungszone (17a) bei starker Vergrößerung eingerichtet ist;
wobei dann, wenn sich der anliegende Ballon (45, 45A, 45C, 45D) im ausgedehnten Zustand befindet:
ein vorderer Oberflächenabschnitt (45b) des anliegenden Ballons (45, 45A, 45C, 45D) dazu eingerichtet ist, sich um die interessierende Zone (17a) anzulegen;
ein hinterer Oberflächenabschnitt (45d) des anliegenden Ballons dazu eingerichtet ist, sich an eine vordere Stirnfläche (33a) des distalen Endabschnitts (18) des ersten Einführabschnitts (10) anzulegen; und
ein an der Frontfläche der Bildaufnahmeeinheit (39) angeordnetes Beobachtungsfenster für die Beobachtung bei starker Vergrößerung in engem Kontakt mit der interessierenden Zone (17a) steht.

2. Endoskopsystem nach Anspruch 1, bei dem der anliegende Ballon (45) aus einem transparenten oder halbtransparenten Material besteht.

3. Endoskopsystem nach Anspruch 1, bei dem der anliegende Ballon (45C) ein zylindrisches Element (36) ist, das aus einem dehnbaren und kompressiblen Schaumgummi besteht.

4. Endoskopsystem nach Anspruch 1, bei dem der anliegende Ballon (45D) mit einem dehnbaren und kompressiblen synthetischem Kautschuk mit einer Mehrzahl Luftkammern (71) ausgebildet ist.

5. Endoskopsystem nach einem der Ansprüche 1 bis 4, bei dem der vordere Oberflächenabschnitt (45b) und der hintere Oberflächenabschnitt (45d) jeweils eine allgemein ebene Form haben, wenn sich der Ballon (45, 45A, 45C, 45D) im ausgedehnten Zustand befindet.

6. Endoskopsystem nach Anspruch 5, bei dem der vordere Oberflächenabschnitt (45b) und der hintere Oberflächenabschnitt (45d) aus einem rutschfesten Element bestehen.

7. Endoskopsystem nach Anspruch 5, bei dem der vordere Oberflächenabschnitt (45b) konkav und konvex ausgebildet ist.

8. Endoskopsystem nach Anspruch 6, bei dem der vordere Oberflächenabschnitt (45b) und der hintere Oberflächenabschnitt (45d) des anliegenden Ballons so ausgebildet sind,
dass sie im ausgedehnten Zustand eine allgemein ebene Form haben, indem die Dicke des vorderen und hinteren Oberflächenabschnitts (45a, 45d) größer ist als die Dicke anderer Abschnitte des anliegenden Ballons, oder indem am vorderen und hinteren Oberflächenabschnitt (45a, 45d) ein weniger dehnbares Material als an anderen Abschnitten des anliegenden Ballons angeordnet ist.

9. Endoskopsystem nach einem der Ansprüche 1 bis 8, bei dem die Position einer Oberfläche des anliegenden Ballons (45), die an der interessierenden Zone (17a) anliegt, in einem Bereich der Schärfentiefe des ersten Endoskops (2) vorgesehen ist.

## Revendications

1. Système d'endoscope (1), comprenant:
un premier endoscope (2) comprenant:
une première partie d'insertion (10) adaptée pour être insérée dans un sujet;
un canal (23) comportant une ouverture (23a) au niveau d'une partie d'extrémité distale (18) de la première partie d'insertion (10);
une fenêtre d'observation (25) au niveau de la partie d'extrémité distale (18) de la première partie d'insertion (10);
une fenêtre d'éclairage (24) adaptée pour éclairer une région d'observation au niveau de la partie d'extrémité distale (18) de la première partie d'insertion (10); et
une unité de capture d'images (27) adaptée pour réaliser une observation normale de la région d'observation (17a);
un deuxième endoscope (3, 3B à D) comportant une deuxième partie d'insertion (37) adaptée pour être insérée dans le canal (23) et enlevée de celui-ci, la deuxième partie d'insertion (37) comprenant une partie d'extrémité distale (35) adaptée pour faire saillie depuis l'ouverture (23a);
un ballon de butée (45, 45A, 45C, 45D) prévu sur une partie périphérique externe de la partie d'extrémité distale (35) de la deuxième partie d'insertion (37) et adapté pour être gonflé et contracté; et
**caractérisé en ce que**
le deuxième endoscope (3, 3B à D) comprend en outre une unité de capture d'images (39) prévue dans la partie d'extrémité distale (18) et adaptée pour réaliser une observation à fort grossissement d'une microstructure histologique d'une région d'intérêt à l'intérieur de la région d'observation (17a);
dans lequel, lorsque le ballon de butée (45, 45A, 45C, 45D) se trouve dans un état gonflé:
une partie de face avant (45b) du ballon de butée (45) est adaptée pour venir en butée autour de la région d'intérêt (17a);
une partie de face arrière (45d) du ballon de butée est adaptée pour venir en butée contre une face d'extrémité avant (33a) de la partie d'extrémité distale (18) de la première partie d'insertion (10); et
une fenêtre d'observation placée au niveau d'une face avant de l'unité de capture d'images (39) pour une observation à fort grossissement est en contact étroit avec la région d'intérêt (17a).

2. Système d'endoscope selon la revendication 1, dans lequel le ballon de butée (45) est constitué d'un matériau transparent ou semi-transparent.

3. Système d'endoscope selon la revendication 1, dans lequel le ballon de butée (45C) est un élément cylindrique (36) constitué d'un caoutchouc mousse dilatable et compressible.

4. Système d'endoscope selon la revendication 1, dans lequel le ballon de butée (45D) est constitué d'un caoutchouc synthétique dilatable et compressible comportant une pluralité de chambres à air (71).

5. Système d'endoscope selon l'une quelconque des revendications 1 à 4, dans lequel, lorsque le ballon (45, 45A, 45C, 45D) se trouve dans l'état gonflé, la partie de face avant (45b) et la partie de face arrière (45d) présentent chacune une forme qui est généralement plane.

6. Système d'endoscope selon la revendication 5, dans lequel la partie de face avant (45b) et la partie de face arrière (45d) sont formées d'un élément antidérapant.

7. Système d'endoscope selon la revendication 5, dans lequel la partie de face avant (45b) est munie d'une concavité et d'une convexité.

8. Système d'endoscope selon la revendication 6, dans lequel la partie de face avant (45b) et la partie de face arrière (45d) du ballon de butée sont formées pour présenter la forme généralement plane dans l'état gonflé en rendant une épaisseur des parties de face avant et arrière (45a, 45d) supérieure à une épaisseur des autres parties du ballon de butée ou en disposant un matériau moins étirable que les autres parties du ballon de butée au niveau des parties de face avant et arrière (45a, 45d).

9. Système d'endoscope selon l'une quelconque des revendications 1 à 8, dans lequel une position d'une surface du ballon de butée (45) venant en butée sur la région d'intérêt (17a) est prévue dans une plage d'une profondeur de champ du premier endoscope (2).
